# EUROPEAN PATENT APPLICATION

(11) **EP 3 922 235 A1**
(43) Date of publication of application: **15.12.2021**
(21) Application number: 20179534.1
(22) Date of filing: 11.06.2020
(51) Int. Cl.: A61K 8/04, A61K 8/34, A61K 8/81, A61K 8/73, A61K 8/92, A61K 8/41, A61Q 17/00

(54) **A HYDROALCOHOLIC GEL AND A METHOD OF MANUFACTURING SAID GEL**

(71) Applicant: Viramal Limited, London W1S 1DN (GB)
(72) Inventor: FIORE, Simona, London, SW6 5AB (GB); BATES, Oliver, London, W8 6QH (GB)
(74) Representative: Holme Patent A/S

(57) **Abstract**

The present invention relates to a hydroalcoholic gel comprising at least 70 weight% alcohol, between 5 and 10 weight% of an oil composition, between 0.15 and 6 weight% of a gelling agent and between than 0.01 and 0.1 weight % of a pH adjusting agent, and wherein the gelling agent consist of between 0.05 and 2.0 weight% of a carbomer and between 0.1 and 4.5 weight% of a cellulose polymer. Said hydroalcoholic gel is unique in that it destroys microorganisms and virus, moisturizes the skin, does not leave an oily residue and a tacky feel on the skin, and adheres firmly to the skin (even after washing) thereby providing a long-term disinfecting effect.

## Description

The present invention relates to a hydroalcoholic gel that both provide a sanitizing effect and a moisturizing benefit to the user's skin.

It is well known that one of the best and easiest ways to defend against harmful bacteria and viruses is washing the hands thoroughly with soap and water for at least 20 seconds. For instance, in order to prevent the spread of severe acute respiratory syndrome coronavirus 2 (SARS-CoV-2), the virus that causes COVID-19, hand hygiene is essential, especially in the absence of a vaccine or effective antiviral drugs.

However, handwashing isn't always practical, especially for healthcare workers. This is due to a lack of access to running water, and a lack of sufficient time to wash the hands thoroughly. Alcohol-based hand sanitizers provide a quick, simple alternative.

It is in this respect generally recommend that consumers use an alcohol-based hand sanitizer that contains at least 60% alcohol, but preferably higher concentrations of alcohol, as such hand sanitizers are effective in inactivating/killing most infectious agents, such as microorganisms and viruses. However, even though alcohol-containing sanitizers are known to possess good sanitizing activity and prevent infections, use of alcohol on the skin at high concentrations often severely dries the skin as the alcohol dissolves the sebum from the skin. Consequently, continuous use of such sanitizers can leave the user's skin dry, often resulting in red, chapped, and cracked skin.

As a result, it is common to restrict the alcohol content in hand sanitizers to about 60% percent. However decreasing the alcohol content in the composition has the unwanted effect of decreasing the composition's potency in killing the infectious agent i.e. bacteria, viruses, fungi, and protozoa.

A problem with the known hand sanitizers is that even though the sanitizer may inactivate/kill most of the infectious agents accumulated on the users hands, the known sanitizers does not have a long lasting effect, as the sanitizer easily is "washed" off, e.g. if the user is out in the rain, sweat, or simply washes his/her hands. Thus, the user has to reapply the hand sanitizer regularly in order to obtain the desired antimicrobial effect, thereby increasing the problems with dryness/redness of the hands.

Accordingly, there is a requirement for an hydroalcoholic gel with a high percentage of alcohol that will effectively kill infectious agents, dry quickly, leave no sticky residue, will not excessively dry the skin and ensure that the composition has a prolonged sanitising effect, even after subsequent hand washing.

These and further aspect are achieved according to the present invention by providing a hydroalcoholic gel comprising at least 70 weight% alcohol, between 5 and 10 weight% of an oil composition, between 0.15 and 6 weight% of a gelling agent and between than 0.01 and 0.1 weight % of a pH adjusting agent, and wherein the gelling agent consist of between 0.05 and 2.0 weight% of a carbomer and between 0.1 and 4.5 weight% of a cellulose polymer.

Said hydroalcoholic gel is unique in that it comprises a high concentration of alcohol, thereby effectively killing microorganisms, virus etc, upon contact, but only low concentrations of other components, such as gelling agents, without compromising the moisturising effect or the stability of the product. In fact the hydroalcoholic gel according to the invention is stable, even after long-term storage. When used, the hydroalcoholic gel destroys microorganisms and virus, moisturizes the skin thereby avoiding red, chapped, and cracked skin; does not leave an oily residue and a tacky feel on the skin, and adheres firmly to the skin (even after washing) thereby providing a long-term disinfecting effect. This prolonged effect may be especially relevant for healthcare workers and/or other groups that are exposed to infections from a variety of sources throughout the course of each day.

It is generally recognised that an alcohol concentration of 70 % by weight (or above) kills/inactivate microorganisms upon contact e.g. by denaturating the proteins in the cell membrane of a bacteria or the proteins in the capsid/envelope of a virus. The alcohol used in the present hydroalcoholic gel may be any kind of alcohol capable of killing/inactivating the infectious agent; it is however preferred that said alcohol is ethanol or isopropanol, or a mixture thereof. For instance, ethanol at concentration at 70% by weight has shown to be a potent virucidal agent inactivating all of the lipophilic viruses (e.g., influenza virus) and many hydrophilic viruses (e.g., rotaviruses).

However, since proteins are denatured more quickly by alcohol in the presence of water, a preferred embodiment of the hydroalcoholic gel according to the invention comprises between 5 - 20 % by weight water, preferably between 12 and 18 % by weight water.

In order to prevent the skin from drying out, due to defattening by the high alcohol concentration, the hydroalcoholic gel according to the invention comprises an oil composition. In this way the hydroalcoholic gel comprises both an aqueous phase and an oily phase, which preferably is combined into a bigel.

A bigel is a uniform semisolid dispersion system that visually appear as a single gel, but in which an oleogel and an aqueous gel, are mixed together e.g. by applying a high shear rate. Bigels are not emulsions, and one of the major advantages of bigels is the improved stability compared to emulsions (water-in-oil and oil-in-water), emulgels, hydrogels and oleogels, which ensures that the hydroalcoholic gel according to the invention remains stable, The enhanced physicochemical stability of the bigels can be attributed to the formation of extra fine colloidal dispersions, which is due to the immobilization of one gel (e.g. the oleogel) in a three-dimension gel network of the other gel (e.g. the aqueous gel). On storage at room temperature, the two components of the bigel do not get separated and the hydroalcoholic gel according to the invention therefore remains stable, even after long term storage, i.e. one year or more.

Further, no separation of the aqueous gel and oleogel is detected when the hydroalcoholic bigel is applied to the skin. Thus, by converting oleogels and hydrogels into bigels, a good patient compliance is provided without compromising the beneficial effects of the individual water and oil phases.

The oil composition in the hydroalcoholic gel is preferably selected from mono-, di-, and triglycerides of synthetic, semi-synthetic and natural origin, and mixtures thereof, e.g. a mixture of capric/caprylic triglycerides.

The synthetic mono-, di- or triglycerides may be Miglyol 810/ 812 (Dynamit Nobel), and the semisynthetic mono-, di-or triglycerides, may be propylene glycol isostearate, such as the product sold under the name "hydrophilol isostearique" (Gattefosse), and the polyglycolysed glyceride "Labrafil^{®} M 1944 CS" (Gattefosse). Mono-, di-or triglycerides of a natural origin, is preferably oils of plant origin, such as sweet almond oil, argan oil and palm oil. It is however preferred that the oil-composition in the present invention is a mixture of capric/caprylic triglycerides such as Labrafac^{®} WL1349 (Gattefosse). Labrafac^{®} WL1349 is a mixture of medium chain Triglycerides, mainly from caprylic (C8) and capric (C10) acids.

When said oil composition is added to the hydroalcoholic gel in a concentration between 5 and 10 weight% of the final product, said hydroalcoholic gel will exhibit both an improved moisturising effect to the skin and prevent the skin from drying out. Furthermore, due to the relatively low concentration of the oil composition said composition will not provide a greasy feeling by leaving a greasy residue on the skin, whereby a high user compliance is provided.

The gelling agent used in the hydroalcoholic gel consists of a combination of a carbomer and a cellulose polymer, in an amount in the final composition which is between 0.15 and 6 % by weight.

Carbomer is a generic name for a family of polymers known as carbopols which are homopolymers of acrylic acid, cross-linked with an allyl ether of pentaerythritol, an allyl ether of sucrose, or an allyl ether of propylene. As a group, they are dry powders with high bulk densities, and form acidic aqueous solutions (pH around 3.0), which thicken at higher pHs (around 5 or 6). They swell in aqueous solution of that pH as much as 1000 times their original volume, and the viscosity of the aqueous phase can therefore be adjusted by adjusting the concentration of the carbomer in the composition and the pH-value of the composition. Preferred examples of such carbomers are Carbopol 974 and Carbopol 980 NF.

Since the viscosity of the carbomer is pH dependent, the carbomer will act as a gelling agent, at least for the aqueous phase of the hydroalcoholic gel, when the pH is adjusted to an optimal value. For the Carbopol-polymers said optimal pH value is between 6.0 and 7.5, which corresponds well with the fact that the antimicrobial composition according to the invention is intended for human topical and/or transdermal use, where it is preferred to provide a composition with a neutral pH-value, i.e. around pH 7. A person skilled in the art will understand that it is not required that a pH-value of 7 is obtained in the composition, only that the pH-value of said composition will not harm and/or irritate the epidermis/skin at the application site.

In order to obtain the optimal pH value e.g. a pH value between 6.0 and 7.5, the pH adjusting agent may be any compound/composition capable of adjusting the pH-value of the composition, e.g. sodium hydroxide (if a higher pH-value is desired) or sorbic acid (if a lower pH value is desired). However, in a preferred embodiment the pH adjusting agent is triethanolamine. The inventors of the present invention have surprisingly found that even though it is generally believed that triethanolamine cannot be used for adjusting the pH value in compositions having an alcohol concentration above 60% by weight small concentrations of triethanolamine in the hydroalcoholic gel composition according to the invention, will both provide the optimal pH-value of between 6.0 and 7.5, the desired viscosity of the product, and a stable product in which the oil phase and water phase will not separate. In a preferred embodiment the pH-adjusting agent is added in an amount between 0.01 and 0.1% by weight, preferably about 0.05 to 0.06 % by weight, based on the weight of the final hydroalcoholic gel according to the invention. Concentrations of triethanolamine above 0.1% by weight based on the final hydroalcoholic gel will result a gel composition which is not substantially homogeneous.

The cellulose polymer used in the gelling agent may be chosen from ethylcellulose, non-sodium carboxy methylcellulose, and mixtures thereof. However, one preferred cellulose polymer is the applicant's gelling agent disclosed in PCT/EP2019/082893, or alternatively the formulation Emulfree^{®} CBG, obtainable from Gattefosse. Said compound contains isostearyl alcohol, butylene glycol cocoate and ethylcellulose. The cellulose polymer gels/thickens especially the oil phase and will therefore aid in providing a stable and homogenous final product. The cellulose polymer is added in an amount between 0.1 and 4.5 weight% of a final hydroalcoholic gel, preferably in an amount between 1 and 4 weight%, e.g. around 2 weight%.

The inventors of the present invention have further found that in order to ensure that the hydroalcoholic gel according to the invention can be used for topical use, i.e. a gel which easily can be applied to the skin, adheres firmly to the skin (even after washing), and does not leave a greasy or messy feeling on the skin, it is preferred that the hydroalcoholic gel has a viscosity between 1000 and 150,000. For the sake of comparison, lotions typically have a viscosity within the range 1,000-30,000 cP (1-30 Pa.s), while creams and ointments have a higher viscosity above 30,000 cP (30 Pa.s), preferably above 80,000 cP (80 Pa.s) and below 150,000 cP (30-150 Pa.s). In a preferred embodiment the hydroalcoholic gel has a viscosity within the range of 30,000-120,000 cP (20-120 Pa.s), preferably around 50,000-80,000 cP (50-80 Pa.s) All viscosities are measured using a Lamy VRM-08 viscometer with an MS DIN module at a temperature of 23°C and at a shear stress of 0.8 s⁻¹.

A person skilled in the art will understand that it is not always necessary to measure the viscosity of the hydroalcoholic gel in order to obtain a gel with the desired viscosity. It can also be determined by feel whether a gel has a viscosity suitable for use as a hand sanitizer. In addition, once the components and conditions for making a gel having a desired viscosity have been determined, it is possible to recreate a gel having that specific viscosity by following the same procedures.

A person skilled in the art can easily obtain a hydroalcoholic gel with any desired viscosity, e.g. by adjusting the concentrations of the gelling agent, i.e. the concentration of the compounds in said agent, the oil composition and/or the pH-adjusting agent.

The inventors of the present invention has found that the hydroalcoholic gel according to the invention is persistent in that it significantly reduces the incidence of infectious agents on skin surfaces for a period of about 3-4 hours, even after subsequent handwashing. Thus, when the gel is applied to the skin, it will not only kill/inactivate microorganisms and vira upon contact, but form a thin film on the skin after application which remains effective for an extended period of time. This film both prevents reinfection on subsequent introduction of infectious agents and functions as a moisturizing layer by slowing moisture loss from the surface of the skin.

Without being bound by theory it is believed that the oil-composition will spread over the skin, and the gelling agent, that will stabilize the oil-composition adding support to the spreading and layering of the oil. The gelling agent may also interact with the user's skin surface, and it is perhaps such a combination of these interactions which results in a very thin physical film layer of the hydroalcoholic gel which resides on the skin surface and provides the prolonged sanitizing and moisturising effect.

In order to further aid in the disinfection properties of the hydroalcoholic gel according to the invention, the gel may in a preferred embodiment further comprise a disinfecting ingredient. Said disinfecting ingredient may be quaternary ammonium, or sodium hypochlorite, but in a preferred embodiment said ingredient is hydrogen peroxide. The disinfecting agent is preferably added to the hydroalcoholic gel in an amount between 0.1 and 6 % by weight.

In combination, the respective compounds of the unique hydroalcoholic gel provide a product having enhanced effectiveness in the inactivation of bacteria and viruses on the skin of a user. The unique relationship between the different compounds, permits the use of relatively low levels of both moisturising agents, and gelling agents, while maintaining a high concentration of alcohol and optionally an disinfection agent, thereby providing a gel which is persistent in its sanitising characteristics, and with both exhibit an enhanced skin-care and stability.

The hydroalcoholic gel is preferably for use as a hand sanitizer, and the inventors of the present invention has found that a preferred hydroalcoholic gel in this respect comprises the following components:

| Components | % by weight of the final gel |
|---|---|
| Purified water | 16.232 |
| 3% H₂O₂ | 4.212 |
| Carbopol^{®} 980 | 0.500 |
| 96% Ethanol | 70.000 |
| Labrafac^{®} WL1349 | 7.000 |
| Emulfree CBG | 2.000 |
| Triethanolamine | 0.056 |

The hydroalcoholic gel according to the invention may in an alternative embodiment comprise at least one active ingredient which may be a pharmaceutical agent.

Orally administered medications are subject to gastrointestinal degradation by acid or enzymes. As the hydroalcoholic gel is intended for topical administration, said gel may be particularly suitable for active pharmaceutical ingredients that are vulnerable to gastrointestinal degradation, such as proteins or peptide therapeutic agents.

Due to the high alcohol concentration in the gel, it is preferred to use an active ingredient which is soluble in the alcohol and which also can be transported across the dermis or epithelium. However, the active ingredient can also be an ingredient which primarily is dissolved/distributed in the oil phase, or the gel can comprise different active ingredients in each phase.

For certain intended uses, the hydroalcoholic gel may comprise the same active ingredient in both the oil phase and the aqueous phase. This is advantageously since the inventors have found that the release profile of the active ingredient into the skin from such a gel, is considerably better than that obtained with a composition based on only one type of gel, oily or aqueous. (Comparisons have been made for a given volume of composition applied and a given concentration of active ingredient relative to the total weight of the composition). This is particularly advantageous when using the gel as an active ingredient reservoir in e.g. a transdermal release system. On the skin, the active ingredient(s) dissolved in the aqueous phase is rapidly taken up by the epidermal layers, which allows the percutaneous passage to start quickly. The active ingredient(s) in the oil phase must first, as a function of its oil/water partition coefficient, pass into the aqueous phase in order to pass into the epidermal layers: this fraction in the oil phase will thus be released with a greater delay, after the active ingredient(s) dissolved in the aqueous gel has been released, thereby providing a sustained release profile.

The present invention also relates to a drug delivery system for an active ingredient. Said drug delivery system is preferably a transdermal release system. However, since the inventors of the present invention has shown that the hydroalcoholic gel does not cause harm and/or irritate neither the epidermis nor the mucosal tissue, despite the high alcohol concentration above 70 % by weight, a mucosal release system for e.g. buccal, nasal, vaginal and rectal administration is also contemplated within the scope of the present invention.

The transdermal and mucosal delivery systems according to the invention has a number of advantages such as overcoming the drawbacks of conventional administration routes, for instance, the direct entry of the drug into the systemic circulation obviates the first pass hepatic effect. In addition, the administration of said delivery systems is generally non-invasive, thereby avoiding the uncomfortable aspects of intravenous, intramuscular, or subcutaneous delivery means. Furthermore, active ingredients can be easily administered and if necessary, removed from the application site. Thus the drug delivery system according to the invention is of value in delivering a growing number of active ingredients (drugs).

Suitable active ingredients may be any suitable compound/composition capable of being delivery through the epidermis or mucosal tissue and may accordingly be hormones; corticoid and corticoid derivatives; dermatological active ingredients; antimicrobial agents; anti-inflammatory agents and wound repair agents.

In a preferred embodiment the hydroalcoholic gel comprises a hormone selected from oestradiol (or other oestrogen), progesterone (or progestins) and testosterone, and said hormone is preferably present in the hydroalcoholic gel at between 0.5 and 2.5 wt% or even more, and more preferably between 0.5 and 1.5 wt%, relative to the total weight of the gel. In a preferred embodiment, the gel comprises 1 wt% hormone, preferably testosterone.

The inventors of the present invention have found that a preferred hydroalcoholic gel in this respect comprises the following components:

| Components | % by weight of the final gel |
|---|---|
| Testosterone | 1.000 |
| Purified water | 13.232 |
| 3% H₂O₂ | 4.212 |
| Carbopol^{®} 980 | 0.500 |
| 96% Ethanol | 70.000 |
| Labrafac^{®} WL1349 | 7.000 |
| Emulfree CBG | 4.000 |
| Triethanolamine | 0.056 |

In a still further preferred embodiment the dermatological active ingredients comprise vitamin E and/or one and more essential oils. Vitamin E is known for having a "moisturizing and healing" effect which helps to "strengthen skin barrier function. Vitamin E is fat-soluble, allowing it to penetrate through the dermis and preserve the lipids. Essential oils are plant extracts made from flowers, leaves, and seeds. The essential oils used in the present invention have properties that among others help to soften dry skin. As examples can be mentioned that chamomile oil contains azulene, known for increasing moisture and reducing inflammation, and sandalwood oil contains compounds known for reducing inflammation while promoting moisture in the skin.

Examples of active ingredients that could be used in the hydroalcoholic gel are shown in table A below:

**Table A**

| |
|---|
| Proteins and peptides, such as GnRH (agonist and antagonist), oxytocin and analogs, somatostatin and analogs, tissue plaminogen activator (TPA), growth hormone releasing hormone (GHRH), corticotropin-releasing hormone (CRH) and analogs, insulin, glucagon like peptide, ghrelin and analogs, follicle-stimulating hormone (FSH), luteinizing hormone (LH), human chorionic gonadotropin (HCG), thyroid-stimulating hormone (TSH), and the like. |
| Steroidal hormones, such as testosterone, dihydrotestosterone (DHT), ethinylestradiol, estrone, estrone sulfate, estradiol, or progestins, such as progesterone, levonorgestrel, desogestrel, Danazol, combinations of sex steroidal hormones, sepranolol and selective progesterone modifiers; and the like |
| Anti-hormones, such as tamoxifen, endoxifen, mifepristone, and the like. |
| Nitrates, such as nitroglycerin, isosorbide, erythrityl tetranitrate, pentaerythritol tetranitrate, and the like. |
| β-adrenergic receptor agonists, such as terbutaline, albuterol, pirbuterol, bitolterol, ritodrine, and the like. |
| β-adrenergic receptor antagonists, such as propranolol, metoprolol, nadolol, atenolol,timolol, esmolol, pindolol, acebutolol, labetalol, and the like. |
| Opioids, such as morphine, hydromorphone, oxymorphone, codeine, hydrocodone, oxycodone, levorphanol, levallorphan, butophanol, buprenorphine, fentanyl, nalbuphine, butorphanol, pentazocine, methadone, etorphine, sufentanil, [D-Ala2, N-MePhe4, Gly-ol]-enkephalin (DAMGO), naloxone, naltrexone, D-Phe-Cys-Tyr-D-Trp-Orn-Thr-Pen-Thr-NH2 (CTOP), diprenorphine, β-funaltrexamine, naloxonazine, nalorphine, and the like. |
| Opioid-antagonists, such as naloxone, nalmefene, and the like. |
| Antidepressants, such as amitriptyline, amoxapine, desipramine, doxepin, imipramine, maprotilen, nortriptyline, protripyline, trimipramine, fluoxetine, trazodone, and the like. |
| HMG CoA reductase inhibitors, such as lovastatin, mevastatin, simvastatin, pravastatin, atorvastatin, and the like. |
| Antihistamines, such as loratadine, chlorpheniramine maleate, brompheniramine maleate, diphenhydramine, dimenhydrinate, carbinoxamine, promethazine, tripelannamine, and the like. |
| ACE inhibitors, such as captopril, enalapril, lisinopril, and the like. |
| Prostaglandins, such as misoprostol and the like. |
| Cannabinoids. |
| Minerals and vitamins, especially iron and vitamin E. |
| Nicotine receptor antagonist, e.g. varenicline, bupropion, and cytisine |
| 5-α-reductase inhibitors |

It is preferred that the hydroalcoholic gel according to the invention is a bigel. Such a bigel is normally obtained by preparing the hydrogels and oleogels individually, after which the two gels are mixed together e.g. by applying a high shear rate. Examples of methods for formulating such bigels are e.g. disclosed in EP1083880 and EP2120865.

However, the inventors of the present invention has found that it is possible to provide the hydroalcoholic gel (bigel) in a single process line, i.e. without having first prepare an aqueous gel and an oleogel, and then combine them.

Thus, the present invention also relates to a method of manufacturing the hydroalcoholic gel according to the invention, said method comprises the following consecutive steps:
a. combine alcohol and optionally water, thereby providing an aqueous solution
b. disperse the carbomer into the aqueous solution , thereby providing a first intermediate composition,
c. mixing the cellulose polymer with the first intermediate composition, thereby providing a second intermediate composition,
d. mixing the oil composition with the second intermediate composition thereby providing a third intermediate composition, and
e. mixing the pH adjusting agent with the third intermediate composition, thereby providing the hydroalcoholic gel.

The thereby obtained gel will have the properties of a bigel, i.e. it is a uniform semisolid system that visually appears as a single gel, have an improved stability compared to an emulsion, and does not leave a greasy or messy feeling on the skin. It is believed that even though the water phase and oil phase are not prepared individually, one gel, (e.g. the oleogel) is immobilized in the three-dimension gel network of the other gel (e.g. the aqueous gel), just as if the two phased were first prepared individually and them mixed together. Thus, the present method provides a simpler and cheaper method for providing the desired hydroalcoholic gel with bigel properties, than the conventional ways of producing a bigel.

In the method according to the invention, the ingredient is added step-wise to the composition, in such a way that each ingredient is added before the next. Each ingredient is preferably mixed with the previously obtained intermediate compositions under vigorous stirring until the composition visually appears homogenous. It is preferred that each intermediate composition is stirred for at least 10 minutes, as this has proven to provide the desired stable gel.

If the hydroalcoholic gel according to the invention comprises a disinfecting agent which is hydrophilic, said agent is preferably mixed with the alcohol and water in step a.

In a similar manner, if the hydroalcoholic gel comprises an active ingredient which can be dissolved in alcohol and/or water, said active ingredient is preferably mixed with the alcohol and water in step a, whereas a hydrophobic active ingredient preferably is mixed with the oil composition in step d, or added to said oil composition prior to adding said composition to the composition obtained in step c.

The obtained hydroalcoholic gel disinfects and provides prolonged sanitizing properties, prevents drying of the skin and will not causing irritation to the skin. In addition to the alcohol, and oil composition the hydroalcoholic gel contains a unique gelling agent and a pH adjusting agent in controlled concentrations thereby providing a unique gel with bigel characteristics. It is believed that it is said characteristics which stabilize the formulation and contribute to elimination of a tacky feel which follows after application of the known sanitizers containing e.g. glycerol.

### Examples.

The general procedure for making the respective compositions for obtaining a hydroalcoholic gel was as follows.

The respective gels were manufactured as follows:
First an aqueous gel was prepared by first combining alcohol, water and in some compositions hydrogen peroxide (the disinfectant), and then disperse the carbomer Carbopol^{®} 980 NF in said mixture under stirring from 2000 to 6000 rpm using a SYLVERSON ^{®} type mixer, thereby providing the aqueous gel.

Then an oleogel was prepared by mixing the oil composition Caprylic/Capric triglycerides Labrafac^{®} WL1349 optionally with a small amount of water (purified water (2) in the following tables) under stirring for 5 minutes using a magnetic stirrer. If Emulfree was part of the composition, the Emulfree was then added under stirring for 10 minutes using a magnet stirrer.

The oleogel was thereafter added to the aqueous gel under quick stirring using an ULTRA TURRAX^{®} type mixer at 13500 rpm for 10 minutes, and the thereby obtained bigel was then transferred to a mortar. In order to adjust the pH-value of the bigel, the pH adjusting agent (sodium hydroxide or triethanolamine) was then added under magnetic stirring.

In order to test which compounds is relevant for providing a gel with a high alcohol concentration, the following three compositions were prepared as described above.

| | **F1** weight% | **F2** weight% | **F3** weight% |
|---|---|---|---|
| **Purified water (1)** | 21.315 | 29.315 | 29.515 |
| **Carbopol^{®} 980** | 1.400 | 1.400 | 1.400 |
| **Ethanol 96%** | 60.000 | 60.000 | 60.000 |
| **Labrafac^{®} WL1349** | 10.000 | 2.000 | 2.000 |
| **Emulfree CBG** | 4.000 | 4.000 | 4.000 |
| **Purified water (2)** | 3.200 | 3.200 | 3.200 |
| **Sodium hydroxide** | 0.085 | 0.085 | 0.085 |
| **Total** | 100.000 | 100.000 | 100.000 |

The compositions F1, F2 and F3 were slightly thick gels with lumps of Carbopol, and it was believed that sodium hydroxide induces the formation of lumps of Carbopol.

In order to evaluate if a pH adjusting agent could be omitted, the following composition were prepared.

| | **F4** weight% |
|---|---|
| **Purified water** | 31.537 |
| **Hydrogen peroxide 30%** | 0.463 |
| **Carbopol^{®} 980** | 0.500 |
| **Ethanol 96%** | 60.000 |
| **Labrafac^{®} WL1349** | 7.000 |
| **Emulfree CBG** | - |
| **Total** | 100.000 |

Formula F4 were a liquid composition with two distinct phases. The oily and aqueous phases were not miscible, and Labrafac (the oil composition) was below the aqueous phase.

In order to test if the oil phase could be combined with the aqueous phase, the F5 composition was formulated. F5 corresponds to F4 but with the addition of the cellulose polymer, Emulfree CBG.

| | **F4** weight% | **F5** weight% |
|---|---|---|
| **Purified water** | 31.537 | 30.428 |
| **Hydrogen peroxide 30%** | 0.463 | 0.447 |
| **Carbopol^{®} 980** | 0.500 | 0.482 |
| **Ethanol 96%** | 60.000 | 57.889 |
| **Labrafac^{®} WL1349** | 7.000 | 6.754 |
| **Emulfree CBG** | - | 4.000 |
| **Total** | 100.000 | 100.000 |

F5 provided a gel, thus it could be concluded that a cellulose polymer, such as Emulfree CBG, were necessary for manufacturing a gel with a high alcohol concentration. However, the obtained gel was not stable, after 5 min the gel separated into two distinct phases.

In order to test if higher concentrations of alcohol could be obtained, formula F6 and F7 were prepared.

| | **F6** weight% | **F7** weight% |
|---|---|---|
| **Purified water** | 18.037 | 18.537 |
| **Hydrogen peroxide 30%** | 0.463 | 0.463 |
| **Carbopol^{®} 980** | 0.500 | - |
| **Ethanol 96%** | 70.000 | 70.000 |
| **Labrafac^{®} WL1349** | 7.000 | 7.000 |
| **Emulfree CBG** | 4.000 | 4.000 |
| **Total** | 100.000 | 100.000 |

These did not result in the formation of a gel as the obtained compositions were liquids. F7 was more liquid than F6. 10 minutes after manufacture, F7 separated into two distinct phases and F6 separated after 1 hour.

Thus, in order to evaluate if a more stable gel could be obtained using a pH adjusting agent, the F6 composition was modified by adding a pH adjusting agent, thereby providing the F8 composition.

| | **F8** weight% |
|---|---|
| **Purified water** | 17.947 |
| **Hydrogen peroxide 30%** | 0.461 |
| **Carbopol^{®} 980** | 0.498 |
| **Ethanol 96%** | 69.650 |
| **Labrafac^{®} WL1349** | 6.965 |
| **Emulfree CBG** | 3.980 |
| **Triethanolamine** | 0.500 |
| **Total** | 100.000 |

Since sodium hydroxide was found to provide gels with lumps of Carbopol the pH adjusting agent triethanolamine were tested. Said agent was used even though it is generally recognised that triethanolamine only is suitable for compositions containing 60 weight% alcohols.

Triethanolamine strongly thickened the solution providing a gel, but the appearance was not homogeneous. In order to evaluate if this was caused by the concentration of triethanolamine, F9, which is a modified composition of F8, was manufactured.

F9 was prepared by adding two drops of triethanolamine to 100 g of the gel with formula F6 under propeller stirring. Surprisingly the obtained gel was homogeneous, slightly thick and was stable for more than 1 day. pH in the composition was 6.0. The resultant composition had the following formula.

| | **F9** weight% |
|---|---|
| **Purified water** | 17.981 |
| **Hydrogen peroxide 30%** | 0.463 |
| **Carbopol^{®} 980** | 0.500 |
| **Ethanol 96%** | 70.000 |
| **Labrafac^{®} WL1349** | 7.000 |
| **Emulfree CBG** | 4.000 |
| **Triethanolamine** | 0.056 |
| **Total** | 100.000 |

Thus, it could be concluded that the hydroalcoholic gel composition could contain only very small concentrations of triethanolamine, preferably not more than 0.1 weight%, but also that said addition provided the desired homogeneous hydroalcoholic gel with bi-gel properties.

In order to evaluate if the manufacturing of both an aqueous gel and an oleogel could be avoided, and instead manufacturing the hydroalcoholic gel in a single continuous process, the following one-line manufacturing process were evaluated.

First alcohol, water and hydrogen peroxide (the disinfectant) were mixed, hereafter was Carbopol^{®} 980 (the carbomer) dispersed into the thereby obtained solution under stirring, and the stirring was maintained for 10 minutes. Emulfree CBG (the cellulose polymer) was then added to the thereby obtained intermediate composition under stirring and the stirring was maintained for 10 minutes. Thereafter Labrafac^{®}WL1349 (the oil composition) was added under stirring, and the stirring maintained for 10 minutes, and finally the pH adjusting agent (triethanolamine) was adding under stirring and said stirring was maintained for 10 minutes.

Simultaneously it was evaluate if the concentration of the cellulose polymer Emulfree CBG could be reduced without compromising the characteristics of the F9 gel using this new manufacturing process and the following two compositions were produced as described above.

| | **F10** weight% | **F11** weight% |
|---|---|---|
| **Purified water** | 14.523 | 14.232 |
| **Hydrogen peroxide 3%** | 4.299 | 4.212 |
| **Carbopol^{®} 980** | 0.510 | 0.500 |
| **Ethanol 96%** | 71.425 | 70.000 |
| **Labrafac^{®} WL1349** | 7.143 | 7.000 |
| **Emulfree CBG** | 2.044 | 4.000 |
| **Triethanolamine** | 0.056 | 0. 056 |
| **Total** | 100.000 | 100.000 |

For the F10 formulation the stirring was performed using a high-performance dispersing machine using the rotor-stator principle.

The F11 was stirred using a mixing machine using the propeller principle.

Both F10 and F11 were visually homogeneous and visually stable for a prolonged period.

It could accordingly be concluded that a hydroalcoholic gel with bigel characteristics could be manufacturing in a single process line, instead of first having to produce an aqueous gel and an oleogel and then combining both, thereby significantly simplifying the manufacturing process.

The concentration of the cellulose polymer, Emulfree, could also be reduced without comprising the appearance, stability and effect of the hydroalcoholic gel according to the invention.

In conclusion, the obtained hydroalcoholic gel, has a high percentage of alcohol, thereby ensuring that said gel effectively will inactivate/kill germs, even after subsequent hand washing. The gel will dry quickly, and will not leave a sticky residue on the skin. At the same time it will exhibit moisturizing effect, thereby ensuring that frequent use of the gel will not excessively dry the skin, and remain stable even after long term storage. Accordingly the hydroalcoholic gel is efficacious for use by both healthcare professionals, patients and other persons interested in preventing the spreading of an infectious disease.

The foregoing description and examples have been set forth merely to illustrate the invention and are not intended to be limiting. Each of the disclosed aspects and embodiments of the invention may be considered individually or in combination with other aspects, embodiments, and variations of the invention.

Modifications and combinations of the above principles and designs are foreseen within the scope of the present invention.

## Claims

1. A hydroalcoholic gel comprising at least 70 weight% alcohol, between 5 and 10 weight% of an oil composition, between 0.15 and 6 weight% of a gelling agent and between than 0.01 and 0.1 weight % of a pH adjusting agent, and wherein the gelling agent consist of between 0.05 and 2.0 weight% of a carbomer and between 0.1 and 4.5 weight% of a cellulose polymer, based on the total weight of the hydroalcoholic gel.

2. The hydroalcoholic gel according to claim 1, wherein said hydroalcoholic gel further comprises between 5 and 20 weight% water, preferably between 12 and 18 weight% water.

3. The hydroalcoholic gel according to claim 1 or 2, wherein the hydroalcoholic gel comprises both an aqueous phase and an oily phase, which is combined into a bigel.

4. The hydroalcoholic gel according to claim 1, 2 or 3 wherein the oil composition in the hydroalcoholic gel is selected from mono-, di-, and triglycerides of synthetic, semi-synthetic and natural origin, and mixtures thereof, preferably a mixture of capric/caprylic triglycerides.

5. The hydroalcoholic gel according to any of the preceding claims, wherein the hydroalcoholic gel has a pH value between 6.0 and 7.5.

6. The hydroalcoholic gel according to any of the preceding claims, wherein the pH adjusting agent is triethanolamine.

7. The hydroalcoholic gel according to claim 6, wherein the amount of triethanolamine in the hydroalcoholic gel is between 0.05 and 0.06 % by weight, based on the total weight of the final hydroalcoholic gel.

8. The hydroalcoholic gel according to any of the preceding claims, wherein the cellulose polymer used is chosen from ethylcellulose, non-sodium carboxy methylcellulose, and mixtures thereof.

9. The hydroalcoholic gel according to any of the preceding claims, wherein the hydroalcoholic gel has a viscosity between 1000 and 150,000 cp, preferably between 50,000 and 80,000 cP as measured using a Lamy VRM-08 viscometer with an MS DIN module at a temperature of 23°C and at a shear stress of 0.8 s⁻¹.

10. The hydroalcoholic gel according to any of the preceding claims, wherein the hydroalcoholic gel further comprises a disinfecting ingredient.

11. The hydroalcoholic gel according to claim 10, wherein said disinfecting ingredient is quaternary ammonium, sodium hypochlorite, hydrogen peroxide or a mixture thereof.

12. The hydroalcoholic gel according to claim 10 or 11, wherein the disinfecting ingredient is added to the hydroalcoholic gel in an amount between 0.1 and 6 % by weight, based on the total weight of the final hydroalcoholic gel.

13. The hydroalcoholic gel according to any of the preceding claims, wherein the hydroalcoholic gel comprise at least one active ingredient.

14. The hydroalcoholic gel according to claim 14, wherein said active ingredient is selected from hormones; corticoid and corticoid derivatives; dermatological active ingredients; antimicrobial agents; anti-inflammatory agents and wound repair agents.

15. The hydroalcoholic gel according to claim 14 or 15, wherein the active ingredient is a hormone, in an amount between 0.5 and 2.5 wt%, more preferably between 0.5 and 1.5 wt%, or even more preferred 1 wt%, based on the total weight of the final hydroalcoholic gel.

16. A method for manufacturing the hydroalcoholic gel according to any of the claims 1 - 15, said method comprises the following consecutive steps:
a. combine alcohol and optionally water, thereby providing an aqueous solution
b. disperse the carbomer into the aqueous solution, thereby providing a first intermediate composition,
c. mixing the cellulose polymer with the first intermediate composition, thereby providing a second intermediate composition,
d. mixing the oil composition with the second intermediate composition thereby providing a third intermediate composition, and
e. mixing the pH adjusting agent with the third intermediate composition, thereby providing the hydroalcoholic gel.

17. The method according to claim 16, wherein each added compound is mixed with the previously obtained solution or intermediate composition under vigorous stirring.

18. The method according to claim 16 or 17, wherein each intermediate composition is stirred for at least 10 minutes.

19. A hand sanitizer comprising the hydroalcoholic gel according to any of the claims 1 - 12.

20. The hand sanitizer according to claim 19, wherein the hydroalcoholic gel comprises 70.0 weight% alcohol, 7.0 weight% of an oil composition; 2.5 weight% of a gelling agent and 0.056 weight % of the pH adjusting agent triethanolamine, and wherein the gelling agent consist of 0.5 weight% of a carbomer and 2.0 weight% of a cellulose polymer, all based on the total weight of the hydroalcoholic gel.

21. The hand sanitizer according to claim 20, wherein the alcohol is ethanol, the oil composition is labrafac WL1349, i.e. a mixture of capric/caprylic triglycerides, the carbomer is Carbopol 980 NF, and the cellulose polymer is Emulfree CBG

22. A transdermal or mucosal delivery system comprising the hydroalcoholic gel according to any of the claims 13 - 15.

23. The delivery system according to claim 22, wherein the delivery system comprises at least 1 weight% testosterone, based on the total weight of the hydroalcoholic gel.

24. The delivery system according to claim 23, wherein the hydroalcoholic gel comprises 1 weight% testosterone, 70.0 weight% alcohol, 7.0 weight% of an oil composition; 4.5 weight% of a gelling agent and 0.056 weight % of the pH adjusting agent triethanolamine, and wherein the gelling agent consist of 0.5 weight% of a carbomer and 4.0 weight% of a cellulose polymer, all based on the total weight of the hydroalcoholic gel.
